# EUROPÄISCHE PATENTANMELDUNG

(11) **EP 3 808 385 A1**
(43) Veröffentlichungstag der Anmeldung: **21.04.2021**
(21) Anmeldenummer: 19203184.7
(22) Anmeldetag: 15.10.2019
(51) Int. Cl.: A61L 24/04, A61L 24/00

(54) **KIT AUS EINEM ZWEI-KOMPONENTEN GEWEBEKLEBER UND EINER TEMPORÄREN SCHUTZABDECKUNG HIERFÜR SOWIE DESSEN VERWENDUNG**

(71) Anmelder: Adhesys Medical GmbH, 52074 Aachen (DE)
(72) Erfinder: Die Erfindernennung liegt noch nicht vor
(74) Vertreter: Davepon, Björn

(57) **Zusammenfassung**

Die vorliegende Erfindung betrifft einen Schichtaufbau zum Stoppen einer Blutung oder des Austritts von Gewebeflüssigkeit aus einer Wunde umfassend eine Gewebekleber-Schicht aus einem wenigstens zwei Komponenten umfassenden Gewebekleber-System und/ oder dessen Umsetzungsprodukten, sowie einer darauf aufgebrachten und die Gewebekleber-Schicht wenigstens teilweise bedeckenden Schutzabdeckung, welche eine nicht antihaftbeschichtete Polymerfolie umfasst, wobei das Gewebekleber-System wenigstens einen aminofunktionellen Asparaginsäureester als Härter mit optional wenigstens einem Füllstoff als erste Komponente und als zweite Komponente ein isocyanatfunktionelles Präpolymer enthält, wobei der Schichtaufbau dadurch gekennzeichnet ist, dass die Polymerkomponente der Polymerfolie ausgewählt ist aus thermoplastischen Polymeren und die Polymerfolie gegenüber dem ausgehärteten Gewebekleber-System eine Abzugskraft im Rollenschälversuch gemäß DIN EN 1464 - 2010 - 06 von weniger als 2,5 N/cm aufweist. Die Erfindung betrifft ferner ein Verfahren zum Stoppen einer Blutung oder des Austritts von Gewebeflüssigkeit aus einer Wunde, ein Kit für ein solches Verfahren sowie die Verwendung einer nicht antihaftbeschichtete Polymerfolie als Schutzabdeckung für ein wenigstens zwei Komponenten umfassendes Gewebekleber-System.

## Beschreibung

Die vorliegende Erfindung betrifft einen Schichtaufbau zum Stoppen einer Blutung oder des Austritts von Gewebeflüssigkeit aus einer Wunde mit einer Gewebekleber-Schicht aus einem wenigstens zwei Komponenten umfassenden Gewebekleber-System und einer die Schicht wenigstens teilweise bedeckenden Schutzabdeckung. Die Erfindung betrifft ferner ein Verfahren zum Stoppen einer Blutung oder des Austritts von Gewebeflüssigkeit aus einer Wunde, ein Kit für ein solches Verfahren sowie die Verwendung einer nicht antihaftbeschichtete Polymerfolie als Schutzabdeckung für ein wenigstens zwei Komponenten umfassendes Gewebekleber-System.

### Stand der Technik:

Aus dem Stand der Technik sind Zweikomponenten-Gewebekleber-Systeme (2K-Gewebekleber-Systeme bekannt. So offenbart die EP 2 699 614 B1 ein 2K-Gewebekleber-System, von denen die eine Komponente ein isocyanatfunktionelles Präpolymer aus einem Polyisocyanat und einem Polyol und die zweite Komponente ein aminofunktioneller Asparaginsäureester ist. Solche Systeme können aufgrund ihrer recht hohen Aushärtungsgeschwindigkeiten nach dem Vermischen der Komponenten unmittelbar auf eine Wunde aufgebracht werden, wo sie an Ort und Stelle in kurzen Zeiträumen zu einem elastischen Klebefilm aushärten, die Wunde verschließen und so die Blutung stoppen. Diese Systeme haben zudem den Vorteil, dass sie im Unterschied zu Gewebeklebstoffen auf Cyanacrylatbasis im menschlichen oder tierischen Körper in Zeiträumen von weniger als einem Jahr biologisch abbaubar sind und keine toxischen Abbauprodukte hierbei entstehen.

Der Gewebekleber wird typischerweise als viskose Masse aus einer Zweikammerspritze auf die Wunde appliziert. Aufgrund seiner hohen Klebrigkeit einerseits und seiner schnellen Aushärtungsgeschwindigkeit andererseits besteht hierbei kaum eine Möglichkeit, den Klebstoff zu verstreichen oder anzudrücken. Entweder würden dabei erhebliche Mengen des Klebstoffs wieder vom Ort der Applikation entfernt oder das Werkzeug zum Verstreichen würde beim Aushärten mit verklebt werden.

In der EP 2 794 710 B1 wird deshalb vorgeschlagen, eine Schutzschicht auf den Klebstoff aufzudrücken. Die Schutzschicht kann eine Metallfolie, eine Kunststofffolie, ein Vlies, ein Gewebe, ein Gelege, ein Gewirke, oder eine Kombination hiervon sein. Diese kann auf dem Klebstoff verbleiben oder zu besseren Ablösbarkeit mit einer antiadhäsiven Beschichtung versehen sein, beispielsweise einer Silikonisierung.

Die vorgeschlagenen Lösungen sind jedoch mit Nachteilen behaftet. Verbleibt die Schutzschicht auf dem Klebstoff, so ist in der Regel die biologische Abbaubarkeit des Schichtverbundes nicht mehr gewährleistet. Beim Einsatz einer antiadhäsiven Beschichtung, beispielsweise auf einer Metallfolie, kann es jedoch zu einer unerwünschten Migration von Bestandteilen der Antihaftbeschichtung, wie einer Silikonisierung, in den Gewebeklebstoff kommen. Hierdurch können unter anderem die Klebstoffeigenschaften negativ beeinflusst werden. Zudem können möglicherweise auch in medizinischer Hinsicht Nachteile hieraus erwachsen.

### Aufgabe:

Die Aufgabe der vorliegenden Erfindung bestand somit darin, ein Schichtsystem aus einem 2K-Gewebekleber-System und einer Folie als Schutzabdeckung zur Verfügung zu stellen, mit dem einerseits starke Blutungen zuverlässig gestoppt und andererseits mögliche Nachteile durch eine Migration von Bestandteilen einer Antihaftbeschichtung in den Klebstoff verhindert werden können.

### Lösung:

Die Aufgabe wird gelöst durch einen Schichtaufbau zum Stoppen einer Blutung oder des Austritts von Gewebeflüssigkeit aus einer Wunde umfassend eine Gewebekleber-Schicht aus einem wenigstens zwei Komponenten umfassenden Gewebekleber-System und/ oder dessen Umsetzungsprodukten, sowie einer darauf aufgebrachten und die Gewebekleber-Schicht wenigstens teilweise bedeckenden Schutzabdeckung, welche eine nicht antihaftbeschichtete Polymerfolie umfasst, wobei das Gewebekleber-System wenigstens einen aminofunktionellen Asparaginsäureester als Härter mit optional wenigstens einem Füllstoff als erste Komponente und als zweite Komponente ein isocyanatfunktionelles Präpolymer enthält, wobei der Schichtaufbau dadurch gekennzeichnet ist, dass die Polymerkomponente der Polymerfolie ausgewählt ist aus thermoplastischen Polymeren und die Polymerfolie gegenüber dem ausgehärteten Gewebekleber-System eine Abzugskraft im Rollenschälversuch gemäß DIN EN 1464 - 2010 - 06 von weniger als 2,5 N/cm aufweist.

Der Erfindung liegt die Erkenntnis zugrunde, dass durch die zusätzliche Abdeckung der Gewebekleberschicht mit einer thermoplastischen Folie während des Aushärtungsvorgangs, der typischerweise nach wenigen Minuten abgeschlossen ist, ein Durchbrechen der Blutung wirksam unterbunden werden kann. Die Folie verhindert hierbei, dass der viskose Klebstoff durch den Blutungsdruck weggeschoben werden kann und es zum Durchbluten kommt. Zugleich ist das Material der Schutzabdeckung so gewählt, dass es nach dem Aushärten des Klebstoffs trotz dessen guter Klebstoffeigenschaften wieder von diesem entfernt werden kann. Die zur Abtrennung erforderliche Abzugskraft von weniger als 1 N/cm ist hierbei geringer als die Adhäsion des Gewebeklebers auf der Haut oder dem Gewebe, sodass beim Abziehen der Schutzabdeckung die Verklebung auf der Haut oder dem Gewebe nicht beeinträchtigt wird. Vorzugsweise ist die zur Abtrennung der Schutzabdeckung erforderliche Abzugskraft um wenigstens 10 % höher als die Abzugskraft des ausgehärteten Gewebekleber-Systems von der Haut oder dem Gewebe gemäß ASTM F 2256 - 05, insbesondere wenigstens 50%, weiter bevorzugt wenigstens 100%.

Als Gewebekleber-System eignen sich beispielsweise solche, wie sie in EP 2 699 614 B1 und in EP 2 794 710 B1 offenbart sind. Die ausgehärteten Gewebekleber sollten in einem Zeitraum von 14 Tagen bis 2 Jahren biologisch abbaubar sein. Die exakte Dauer der Abbaubarkeit wird bei einer Klebstoffformulierung typischerweise von dem biologischen Gewebe beeinflusst, auf dem diese aufgebracht ist, und zwar insbesondere vom Grad des Stoffwechsels an der Verklebungsstelle. Dabei fördert ein hoher Stoffwechsel die Geschwindigkeit des biologischen Abbaus. Ferner soll das ausgehärtete System nach ISO 10993 bei der Anwendung im Menschen keine Zytotoxizität aufweisen.

Besonders bevorzugt sind die Gewebekleber-Systeme aus den Beispielen der EP 2 794 710 B1 basierend auf Polyol 1, welches zum Präpolymer 2 umgesetzt wird und dann jeweils mit den Aspartaten A und B sowie PEG 200 in den folgenden Mischungsverhältnissen formuliert wird:

| | |
|---|---|
| Aspartat A/Aspartat B/PEG 200 | 0,369/0,098/0,53 |
| Aspartat A/Aspartat B/PEG 200 | 0,29/0, 175/0,55 |

Vorteilhafterweise ist das Gewebekleber-System derart gewählt, dass der ausgehärtete Gewebekleber eine Abzugskraft von der Haut oder dem Gewebe gemäß ASTM F 2256 - 05 von wenigstens 2,5 N/cm aufweist, insbesondere von wenigstens 2,8 N/cm.

Nach einer bevorzugten Weiterbildung des erfindungsgemäßen Schichtaufbaus deckt die Schutzabdeckung die Gewebekleber-Schicht zumindest vollflächig ab oder überragt diese wenigstens teilweise seitlich. Besonders bevorzugt überragt die Schutzabdeckung die Gewebekleber-Schicht seitlich (vollständig) umlaufend. Dies hat neben der eigentlichen Verbesserung des Schichtaufbaus im Hinblick auf das Stoppen der Blutung den weiteren Vorteil, dass der Anwender beim Andrücken der Folie nicht mit dem Klebstoff in Kontakt kommt.

Vorzugsweise weist die Polymerfolie eine Reißdehnung von 25 bis 1000%, gemessen nach ISO 527-3 - 2019-02 auf, insbesondere von 50 bis 700%. Dies ist bevorzugt weil durch diese Dehnbarkeit die Ablösbarkeit der Folie gegenüber dem Gewebekleber erleichtert wird, da eine Dehnung der Folie während des Abziehens an der Phasengrenze Folie/ Klebstoff den adhäsiven Bruch erleichtert.

Die Dicke der eingesetzten Polymerfolie kann in weiten Bereichen variiert werden, wobei eine Schichtdicke von 20 bis 400 µm, insbesondere von 50 bis 250 µm, bevorzugt ist.

Erfindungsgemäß weist die Polymerfolie gegenüber dem ausgehärteten Gewebekleber-System eine Abzugskraft im Rollenschälversuch gemäß DIN EN 1464 - 2010 - 06 von weniger als 2,5 N/cm auf. Insbesondere besitzt die Polymerfolie gegenüber dem ausgehärteten Gewebekleber-System eine Abzugskraft im Rollenschälversuch gemäß DIN EN 1464 - 2010 - 06 von 0,01 bis 2,0 N/cm, bevorzugt von 0,05 bis 1,5 N/cm, weiter bevorzugt von 0,1 bis 1,2 N/cm, besonders bevorzugt von 0,2 bis 1,0 N/cm. Dies ist besonders bevorzugt, weil durch die niedrigen Abzugskräfte der Patient das Abziehen der Folie kaum bemerkt und es ihm insbesondere keine Schmerzen bereitet. Zudem werden hierdurch weitere Traumata oder Schäden am Gewebe vermieden.

Das Polymer der Polymerfolie kann beispielsweise ausgewählt sein aus Polyethersulfonen, Polysulfonen, Polyolefinen, Polyestern, PTFE, Silikonen oder Mischpolymeren und/oder Mischungen von diesen, insbesondere Polyethylen. Bevorzugt handelt es sich um eine einschichtige Polymerfolie. Dies ist deshalb bevorzugt, weil mehrschichtige Folien nicht immer stabil gegenüber hochenergetischer Strahlung sind, die häufig zur medizinischen Sterilisation eingesetzt wird und dann zur Delamination neigen. Ganz besonders bevorzugt ist eine Polymerfolie, die Polyethylen niedriger Dichte (LDPE) als Polymer enthält und insbesondere zu mehr als 99 Gew.-% bezogen auf das Folienmaterial daraus besteht, insbesondere zu mehr als 99,9 Gew.-%, oder gar zu mehr als 99,99 Gew.-% daraus besteht.

Der erfindungsgemäße Schichtaufbau umfasst keine antihaftbeschichtete Folie. Die Polymerfolie weist insbesondere keine Silikonisierung auf.

Die Erfindung betrifft weiterhin ein Verfahren zum Stoppen einer Blutung oder des Austritts von Gewebeflüssigkeit aus einer Wunde, wobei das Verfahren die folgenden Schritte umfasst:
a) Vermischen der Komponenten eines wenigstens zwei Komponenten umfassenden Gewebekleber-Systems, das wenigstens einen aminofunktionellen Asparaginsäureester als Härter mit optional wenigstens einem Füllstoff als erste Komponente und als zweite Komponente ein isocyanatfunktionelles Präpolymer umfasst;
b) Auftragen des im Schritt a) erzeugten Gemisches auf die Wunde, wobei eine Gewebekleberschicht erzeugt wird;
c) wenigstens teilweises Bedecken der Gewebekleberschicht mit einer Schutzabdeckung, die eine nicht antihaftbeschichtete Polymerfolie umfasst, dessen Polymerkomponente ausgewählt ist aus thermoplastischen Polymeren, wobei die Polymerfolie gegenüber dem ausgehärteten Gewebekleber-System eine Abzugskraft im Rollenschälversuch gemäß DIN EN 1464 - 2010 - 06 von weniger als 2,5 N/cm aufweist;
d) Aushärtenlassen des Gewebekleber-Systems;
e) Optional Entfernen der Schutzabdeckung nach Überschreiten des Gelpunktes des Gewebeklebersystems oder nach dessen vollständigem Aushärten.

Der Gelpunkt des Gewebeklebers charakterisiert den Phasenübergang während des Aushärteprozesses von flüssig nach fest und ist im Rahmen der Erfindung dahingehend definiert, dass bei der rheologischen Untersuchung der Speichermodul betragsgleich dem Verlustmodul ist.

Die Erfindung betrifft zudem ein Verfahren zum Stoppen einer Blutung oder des Austritts von Gewebeflüssigkeit aus einer Wunde, wobei das Verfahren die folgenden Schritte umfasst:
a) Vermischen der Komponenten eines wenigstens zwei Komponenten umfassenden Gewebekleber-Systems, das wenigstens einen aminofunktionellen Asparaginsäureester als Härter mit optional wenigstens einem Füllstoff als erste Komponente und als zweite Komponente ein isocyanatfunktionelles Präpolymer umfasst;
b) Auftragen des im Schritt a) erzeugten Gemisches auf eine Schutzabdeckung unter Erzeugung eines Schichtaufbaus, wobei die eine nicht antihaftbeschichtete Polymerfolie umfasst, dessen Polymerkomponente ausgewählt ist aus thermoplastischen Polymeren, wobei die Polymerfolie gegenüber dem ausgehärteten Gewebekleber-System eine Abzugskraft im Rollenschälversuch gemäß DIN EN 1464 - 2010 - 06 von weniger als 2,5 N/cm aufweist;
c) Auflegen des Schichtaufbaus mit der Seite der Gewebekleberschicht auf die Wunde und gewünschtenfalls Andrücken;
d) Aushärtenlassen des Gewebeklebersystems;
e) Optional Entfernen der Schutzabdeckung nach Überschreiten des Gelpunktes des Gewebeklebersystems oder nach dessen vollständigem Aushärten.

Das Auftragen des im Schritt a) erzeugten Gemisches auf die Schutzabdeckung erfolgt zweckmäßigerweise einseitig.

Bei den beiden vorstehenden erfindungsgemäßen Verfahrensvarianten nach Anspruch 10 oder 11, dadurch gekennzeichnet, dass die Schutzabdeckung im Schritt e) nach frühestens 30 Sekunden entfernt wird, insbesondere nach 30 Sekunden bis 10 Minuten .

Vorzugsweise ist eine nach einem erfindungsgemäßen Verfahren erzeugte Verklebung mit dem ausgehärteten Gewebeklebersystem derart ausgestaltet, dass diese einem Blutdruck von wenigstens 120 mmHg standhält.

Ein weiterer Gegenstand der vorliegenden Erfindung betrifft ein Kit umfassend ein wenigstens zwei Komponenten umfassendes Gewebekleber-System und eine Schutzabdeckung hierfür, sowie optional eine Verfahrensanleitung zum Verschließen einer Wunde mithilfe des Kits, insbesondere einer Verfahrensanleitung, die ein erfindungsgemäßes Verfahren beschreibt, wobei das Gewebekleber-System wenigstens einen aminofunktionellen Asparaginsäureester als Härter mit optional wenigstens einem Füllstoff als erste Komponente und als zweite Komponente ein isocyanatfunktionelles Präpolymer enthält und wobei die Schutzabdeckung eine nicht antihaftbeschichtete Polymerfolie umfasst, dessen Polymerkomponente ausgewählt ist aus thermoplastischen Polymeren, wobei die Polymerfolie gegenüber dem ausgehärteten Gewebekleber-System eine Abzugskraft im Rollenschälversuch gemäß DIN EN 1464 - 2010 - 06 von weniger als 2,5 N/cm aufweist.

Die Erfindung betrifft weiterhin die Verwendung einer nicht antihaftbeschichtete Polymerfolie als Schutzabdeckung, insbesondere als temporäre Schutzabdeckung, für ein wenigstens zwei Komponenten umfassendes Gewebekleber-System und/ oder dessen Umsetzungsprodukte, wobei das Gewebekleber-System wenigstens einen aminofunktionellen Asparaginsäureester als Härter mit optional wenigstens einem Füllstoff als erste Komponente und als zweite Komponente ein isocyanatfunktionelles Präpolymer enthält, wobei die Polymerkomponente der Polymerfolie ausgewählt ist aus thermoplastischen Polymeren und die Polymerfolie gegenüber dem ausgehärteten Gewebekleber-System eine Abzugskraft im Rollenschälversuch gemäß DIN EN 1464 - 2010 - 06 von weniger als 2,5 N/cm aufweist.

Die Erfindung betrifft insbesondere die folgenden Ausführungsformen:
Nach einer ersten Ausführungsform betrifft die Erfindung einen Schichtaufbau zum Stoppen einer Blutung oder des Austritts von Gewebeflüssigkeit aus einer Wunde umfassend eine Gewebekleber-Schicht aus einem wenigstens zwei Komponenten umfassenden Gewebekleber-System und/ oder dessen Umsetzungsprodukten, sowie einer darauf aufgebrachten und die Gewebekleber-Schicht wenigstens teilweise bedeckenden Schutzabdeckung, welche eine nicht antihaftbeschichtete Polymerfolie umfasst, wobei das Gewebekleber-System wenigstens einen aminofunktionellen Asparaginsäureester als Härter mit optional wenigstens einem Füllstoff als erste Komponente und als zweite Komponente ein isocyanatfunktionelles Präpolymer enthält, dadurch gekennzeichnet, dass die Polymerkomponente der Polymerfolie ausgewählt ist aus thermoplastischen Polymeren und die Polymerfolie gegenüber dem ausgehärteten Gewebekleber-System eine Abzugskraft im Rollenschälversuch gemäß DIN EN 1464 - 2010 - 06 von weniger als 2,5 N/cm aufweist.

Nach einer zweiten Ausführungsform betrifft die Erfindung einen Schichtaufbau nach Ausführungsform 1, dadurch gekennzeichnet, dass die Schutzabdeckung die Gewebekleber-Schicht zumindest vollflächig abdeckt oder wenigstens teilweise seitlich überragt, wobei die Schutzabdeckung die Gewebekleber-Schicht insbesondere seitlich umlaufend überragt.

Nach einer dritten Ausführungsform betrifft die Erfindung einen Schichtaufbau nach Ausführungsform 1 oder 2, dadurch gekennzeichnet, dass die Polymerfolie eine Reißdehnung von 25 bis 1000%, gemessen nach ISO 527-3 - 2019-02 aufweist, insbesondere von 50 bis 700%.

Nach einer vierten Ausführungsform betrifft die Erfindung einen Schichtaufbau nach einer der vorstehenden Ausführungsformen, dadurch gekennzeichnet, dass die Polymerfolie eine Schichtdicke von 20 bis 400 µm aufweist, insbesondere von 50 bis 250 µm.

Nach einer fünften Ausführungsform betrifft die Erfindung einen Schichtaufbau nach einer der vorstehenden Ausführungsformen, dadurch gekennzeichnet, dass die Polymerfolie gegenüber dem ausgehärteten Gewebekleber-System eine Abzugskraft im Rollenschälversuch gemäß DIN EN 1464 - 2010 - 06 von 0,01 bis 2,0 N/cm, bevorzugt von 0,05 bis 1,5 N/cm, weiter bevorzugt von 0,1 bis 1,2 N/cm, besonders bevorzugt von 0,2 bis 1,0 N/cm.

Nach einer sechsten Ausführungsform betrifft die Erfindung einen Schichtaufbau nach einer der vorstehenden Ausführungsformen, dadurch gekennzeichnet, dass das Polymer der Polymerfolie ausgewählt ist aus Polyethersulfonen, Polysulfonen, Polyolefinen, Polyestern, PTFE, Silikonen oder Mischpolymeren und/oder Mischungen von diesen, insbesondere Polyethylen.

Nach einer siebten Ausführungsform betrifft die Erfindung einen Schichtaufbau nach Ausführungsform 6, dadurch gekennzeichnet, dass die Polymerfolie bevorzugt Polyethylen niedriger Dichte (LDPE) als Polymer enthält und insbesondere zu mehr als 99 Gew.-% bezogen auf das Folienmaterial daraus besteht, insbesondere zu mehr als 99,9 Gew.-% .

Nach einer achten Ausführungsform betrifft die Erfindung einen Schichtaufbau nach einer der vorstehenden Ausführungsformen, dadurch gekennzeichnet, dass die Polymerfolie keine Silikonisierung aufweist.

Nach einer neunten Ausführungsform betrifft die Erfindung ein Verfahren zum Stoppen einer Blutung oder des Austritts von Gewebeflüssigkeit aus einer Wunde, wobei das Verfahren die folgenden Schritte umfasst:
a) Vermischen der Komponenten eines wenigstens zwei Komponenten umfassenden Gewebekleber-Systems, das wenigstens einen aminofunktionellen Asparaginsäureester als Härter mit optional wenigstens einem Füllstoff als erste Komponente und als zweite Komponente ein isocyanatfunktionelles Präpolymer umfasst;
b) Auftragen des im Schritt a) erzeugten Gemisches auf die Wunde, wobei eine Gewebekleberschicht erzeugt wird;
c) wenigstens teilweises Bedecken der Gewebekleberschicht mit einer Schutzabdeckung, die eine nicht antihaftbeschichtete Polymerfolie umfasst, dessen Polymerkomponente ausgewählt ist aus thermoplastischen Polymeren, wobei die Polymerfolie gegenüber dem ausgehärteten Gewebekleber-System eine Abzugskraft im Rollenschälversuch gemäß DIN EN 1464 - 2010 - 06 von weniger als 2,5 N/cm aufweist;
d) Aushärtenlassen des Gewebekleber-Systems;
e) Optional Entfernen der Schutzabdeckung nach Überschreiten des Gelpunktes des Gewebeklebersystems oder nach dessen vollständigem Aushärten.

Nach einer zehnten Ausführungsform betrifft die Erfindung ein Verfahren zum Stoppen einer Blutung oder des Austritts von Gewebeflüssigkeit aus einer Wunde, wobei das Verfahren die folgenden Schritte umfasst:
a) Vermischen der Komponenten eines wenigstens zwei Komponenten umfassenden Gewebekleber-Systems, das wenigstens einen aminofunktionellen Asparaginsäureester als Härter mit optional wenigstens einem Füllstoff als erste Komponente und als zweite Komponente ein isocyanatfunktionelles Präpolymer umfasst;
b) Auftragen des im Schritt a) erzeugten Gemisches auf eine Schutzabdeckung unter Erzeugung eines Schichtaufbaus, wobei die eine nicht antihaftbeschichtete Polymerfolie umfasst, dessen Polymerkomponente ausgewählt ist aus thermoplastischen Polymeren, wobei die Polymerfolie gegenüber dem ausgehärteten Gewebekleber-System eine Abzugskraft im Rollenschälversuch gemäß DIN EN 1464 - 2010 - 06 von weniger als 2,5 N/cm aufweist;
c) Auflegen des Schichtaufbaus mit der Seite der Gewebekleberschicht auf die Wunde und gewünschtenfalls Andrücken;
d) Aushärtenlassen des Gewebeklebersystems;
e) Optional Entfernen der Schutzabdeckung nach Überschreiten des Gelpunktes des Gewebeklebersystems oder nach dessen vollständigem Aushärten.

Nach einer elften Ausführungsform betrifft die Erfindung ein Verfahren nach Ausführungsform 9 oder 10, dadurch gekennzeichnet, dass die Schutzabdeckung im Schritt e) nach frühestens 30 Sekunden entfernt wird, insbesondere nach 30 Sekunden bis 10 Minuten .

Nach einer zwölften Ausführungsform betrifft die Erfindung ein Verfahren nach einer der Ausführungsformen 9 bis 11, dadurch gekennzeichnet, dass die Verklebung mit dem ausgehärteten Gewebeklebersystem einem Blutdruck von wenigstens 120 mmHg standhält.

Nach einer dreizehnten Ausführungsform betrifft die Erfindung ein Kit umfassend ein wenigstens zwei Komponenten umfassendes Gewebekleber-System und eine Schutzabdeckung hierfür, sowie optional eine Verfahrensanleitung zum Verschließen einer Wunde mithilfe des Kits, insbesondere einer Verfahrensanleitung, die das Verfahren gemäß Anspruch 10 und/ oder 11 sowie gewünschtenfalls 11 und/ oder 12 beschreibt, wobei das Gewebekleber-System wenigstens einen aminofunktionellen Asparaginsäureester als Härter mit optional wenigstens einem Füllstoff als erste Komponente und als zweite Komponente ein isocyanatfunktionelles Präpolymer enthält und wobei die Schutzabdeckung eine nicht antihaftbeschichtete Polymerfolie umfasst, dessen Polymerkomponente ausgewählt ist aus thermoplastischen Polymeren, wobei die Polymerfolie gegenüber dem ausgehärteten Gewebekleber-System eine Abzugskraft im Rollenschälversuch gemäß DIN EN 1464 - 2010 - 06 von weniger als 2,5 N/cm aufweist.

Nach einer vierzehnten Ausführungsform betrifft die Erfindung die Verwendung einer nicht antihaftbeschichtete Polymerfolie als Schutzabdeckung, insbesondere als temporäre Schutzabdeckung, für ein wenigstens zwei Komponenten umfassendes Gewebekleber-System und/ oder dessen Umsetzungsprodukte, wobei das Gewebekleber-System wenigstens einen aminofunktionellen Asparaginsäureester als Härter mit optional wenigstens einem Füllstoff als erste Komponente und als zweite Komponente ein isocyanatfunktionelles Präpolymer enthält, wobei die Polymerkomponente der Polymerfolie ausgewählt ist aus thermoplastischen Polymeren und die Polymerfolie gegenüber dem ausgehärteten Gewebekleber-System eine Abzugskraft im Rollenschälversuch gemäß DIN EN 1464 - 2010 - 06 von weniger als 2,5 N/cm aufweist.

### Beispiel:

Die vorliegende Erfindung wird im Folgenden anhand eines Ausführungsbeispiels näher erläutert.

### Eingesetzte Materialien:

Gewebekleber 1: Zwei-Komponenten Gewebekleber basierend auf dem Polyol 1 der EP 2 794 710 B1, welches zum Präpolymer 2 umgesetzt wird und dann jeweils mit den Aspartaten A und B sowie PEG 200 im folgenden Mischungsverhältnis (Aspartat A/Aspartat B/PEG 200; 0,369/0,098/0,53) formuliert wird.
Ledersubstrat: Leder aus Schweinehaut, Wet White Gerbung, Dicke 1,2 mm, geruchslos
Folie: LDPE-Folie nicht antihaftbeschichtet, Dicke 80 µm

### Messung der Abzugskraft:

Zur Bestimmung der Abzugskraft wurde zunächst das Ledersubstrat in den Maßen 150x25 mm mit einem handelsüblichen Cyanacrylatklebstoff (bspw. Loctite ® 401) auf eine Edelstahlplatte geklebt. Auf die der Edelstahlplatte gegenüberliegende Lederoberfläche wurde der Gewebekleber 1 unmittelbar nach dem Vermischen der zwei Komponenten in einer ca. 1 mm dicken Schicht flächig aufgetragen. Im Anschluss wurde die Oberfläche des applizierten Gewebeklebers zügig mit der nicht antihaftbeschichteten LDPE-Folie abgedeckt und für ca. 10 Sekunden leicht angedrückt. Nach Erreichen des Gelpunktes des Gewebeklebers 1 nach ca. 100 Sekunden wurde der Schichtverbund in eine Rollenschälprüfmaschine eingespannt und die Abzugskraft der PE-Folie im Rollenschälversuch gemäß DIN EN 1464 - 2010 - 06 bestimmt, wobei die Abzugskraft unterhalb von 2,5 N/cm lag.

### Simulierte Anwendung des erfindungsgemäßen Schichtverbundes auf einer Gewebeprobe:

Die zwei Komponenten des Gewebeklebers 1 wurden vermischt und mittels eines Applikators unmittelbar danach in ca. 1 mm Schichtdicke flächig auf ein mit Wasser angefeuchtetes Steak als Gewebeprobe aufgetragen. Im Anschluss wurde die Oberfläche des applizierten Gewebeklebers zügig mit derselben nicht antihaftbeschichteten PE-Folie wie beim vorstehenden Versuch abgedeckt und für ca. 10 Sekunden leicht angedrückt. Nach Erreichen des Gelpunktes nach ca. 100 Sekunden wurde die PE-Folie händisch unter Verwendung eines mechanischen Kraftmessers ungefähr im 90° Winkel zum Steak vom Gewebekleber abgezogen, wobei sich die Folie leicht abziehen ließ. Der Kraftmesser zeigte hierbei einen Wert von höchstens 0,6 N/cm an. Der applizierte Gewebekleber löste sich dabei nicht ab und verblieb vollständig auf dem Steak.

## Patentansprüche

1. Schichtaufbau zum Stoppen einer Blutung oder des Austritts von Gewebeflüssigkeit aus einer Wunde umfassend eine Gewebekleber-Schicht aus einem wenigstens zwei Komponenten umfassenden Gewebekleber-System und/ oder dessen Umsetzungsprodukten, sowie einer darauf aufgebrachten und die Gewebekleber-Schicht wenigstens teilweise bedeckenden Schutzabdeckung, welche eine nicht antihaftbeschichtete Polymerfolie umfasst, wobei das Gewebekleber-System wenigstens einen aminofunktionellen Asparaginsäureester als Härter mit optional wenigstens einem Füllstoff als erste Komponente und als zweite Komponente ein isocyanatfunktionelles Präpolymer enthält,
**dadurch gekennzeichnet, dass**
die Polymerkomponente der Polymerfolie ausgewählt ist aus thermoplastischen Polymeren und die Polymerfolie gegenüber dem ausgehärteten Gewebekleber-System eine Abzugskraft im Rollenschälversuch gemäß DIN EN 1464 - 2010 - 06 von weniger als 2,5 N/cm aufweist.

2. Schichtaufbau nach Anspruch 1, **dadurch gekennzeichnet, dass** die Schutzabdeckung die Gewebekleber-Schicht zumindest vollflächig abdeckt oder wenigstens teilweise seitlich überragt, wobei die Schutzabdeckung die Gewebekleber-Schicht insbesondere seitlich umlaufend überragt.

3. Schichtaufbau nach Anspruch 1 oder 2, **dadurch gekennzeichnet, dass** die Polymerfolie eine Reißdehnung von 25 bis 1000%, gemessen nach ISO 527-3 - 2019-02 aufweist, insbesondere von 50 bis 700%.

4. Schichtaufbau nach einem der vorstehenden Ansprüche, **dadurch gekennzeichnet, dass** die Polymerfolie eine Schichtdicke von 20 bis 400 µm aufweist, insbesondere von 50 bis 250 µm.

5. Schichtaufbau nach einem der vorstehenden Ansprüche, **dadurch gekennzeichnet, dass** die Polymerfolie gegenüber dem ausgehärteten Gewebekleber-System eine Abzugskraft im Rollenschälversuch gemäß DIN EN 1464 - 2010 - 06 von 0,01 bis 2,0 N/cm, bevorzugt von 0,05 bis 1,5 N/cm, weiter bevorzugt von 0,1 bis 1,2 N/cm, besonders bevorzugt von 0,2 bis 1,0 N/cm.

6. Schichtaufbau nach einem der vorstehenden Ansprüche, **dadurch gekennzeichnet, dass** das Polymer der Polymerfolie ausgewählt ist aus Polyethersulfonen, Polysulfonen, Polyolefinen, Polyestern, PTFE, Silikonen oder Mischpolymeren und/oder Mischungen von diesen, insbesondere Polyethylen.

7. Schichtaufbau gemäß Anspruch 6, **dadurch gekennzeichnet, dass** die Polymerfolie bevorzugt Polyethylen niedriger Dichte (LDPE) als Polymer enthält und insbesondere zu mehr als 99 Gew.-% bezogen auf das Folienmaterial daraus besteht, insbesondere zu mehr als 99,9 Gew.-% .

8. Schichtaufbau nach einem der vorstehenden Ansprüche, **dadurch gekennzeichnet, dass** die Polymerfolie keine Silikonisierung aufweist.

9. Verfahren zum Stoppen einer Blutung oder des Austritts von Gewebeflüssigkeit aus einer Wunde, wobei das Verfahren die folgenden Schritte umfasst:
a) Vermischen der Komponenten eines wenigstens zwei Komponenten umfassenden Gewebekleber-Systems, das wenigstens einen aminofunktionellen Asparaginsäureester als Härter mit optional wenigstens einem Füllstoff als erste Komponente und als zweite Komponente ein isocynatfunktionelles Präpolymer umfasst;
b) Auftragen des im Schritt a) erzeugten Gemisches auf die Wunde, wobei eine Gewebekleberschicht erzeugt wird;
c) wenigstens teilweises Bedecken der Gewebekleberschicht mit einer Schutzabdeckung, die eine nicht antihaftbeschichtete Polymerfolie umfasst, dessen Polymerkomponente ausgewählt ist aus thermoplastischen Polymeren, wobei die Polymerfolie gegenüber dem ausgehärteten Gewebekleber-System eine Abzugskraft im Rollenschälversuch gemäß DIN EN 1464 - 2010 - 06 von weniger als 2,5 N/cm aufweist;
d) Aushärtenlassen des Gewebekleber-Systems;
e) Optional Entfernen der Schutzabdeckung nach Überschreiten des Gelpunktes des Gewebeklebersystems oder nach dessen vollständigem Aushärten.

10. Verfahren zum Stoppen einer Blutung oder des Austritts von Gewebeflüssigkeit aus einer Wunde, wobei das Verfahren die folgenden Schritte umfasst:
a) Vermischen der Komponenten eines wenigstens zwei Komponenten umfassenden Gewebekleber-Systems, das wenigstens einen aminofunktionellen Asparaginsäureester als Härter mit optional wenigstens einem Füllstoff als erste Komponente und als zweite Komponente ein isocyanatfunktionelles Präpolymer umfasst;
b) Auftragen des im Schritt a) erzeugten Gemisches auf eine Schutzabdeckung unter Erzeugung eines Schichtaufbaus, wobei die eine nicht antihaftbeschichtete Polymerfolie umfasst, dessen Polymerkomponente ausgewählt ist aus thermoplastischen Polymeren, wobei die Polymerfolie gegenüber dem ausgehärteten Gewebekleber-System eine Abzugskraft im Rollenschälversuch gemäß DIN EN 1464 - 2010 - 06 von weniger als 2,5 N/cm aufweist;
c) Auflegen des Schichtaufbaus mit der Seite der Gewebekleberschicht auf die Wunde und gewünschtenfalls Andrücken;
d) Aushärtenlassen des Gewebeklebersystems;
e) Optional Entfernen der Schutzabdeckung nach Überschreiten des Gelpunktes des Gewebeklebersystems oder nach dessen vollständigem Aushärten.

11. Verfahren nach Anspruch 9 oder 10, **dadurch gekennzeichnet, dass** die Schutzabdeckung im Schritt e) nach frühestens 30 Sekunden entfernt wird, insbesondere nach 30 Sekunden bis 10 Minuten.

12. Verfahren nach einem der Ansprüche 9 bis 11, **dadurch gekennzeichnet, dass** die Verklebung mit dem ausgehärteten Gewebeklebersystem einem Blutdruck von wenigstens 120 mmHg standhält.

13. Kit umfassend ein wenigstens zwei Komponenten umfassendes Gewebekleber-System und eine Schutzabdeckung hierfür, sowie optional eine Verfahrensanleitung zum Verschließen einer Wunde mithilfe des Kits, insbesondere einer Verfahrensanleitung, die das Verfahren gemäß Anspruch 9 und/ oder 10 sowie gewünschtenfalls 11 und/ oder 12 beschreibt, wobei das Gewebekleber-System wenigstens einen aminofunktionellen Asparaginsäureester als Härter mit optional wenigstens einem Füllstoff als erste Komponente und als zweite Komponente ein isocyanatfunktionelles Präpolymer enthält und wobei die Schutzabdeckung eine nicht antihaftbeschichtete Polymerfolie umfasst, dessen Polymerkomponente ausgewählt ist aus thermoplastischen Polymeren, wobei die Polymerfolie gegenüber dem ausgehärteten Gewebekleber-System eine Abzugskraft im Rollenschälversuch gemäß DIN EN 1464 - 2010 - 06 von weniger als 2,5 N/cm aufweist.

14. Verwendung einer nicht antihaftbeschichtete Polymerfolie als Schutzabdeckung, insbesondere als temporäre Schutzabdeckung, für ein wenigstens zwei Komponenten umfassendes Gewebekleber-System und/ oder dessen Umsetzungsprodukte, wobei das Gewebekleber-System wenigstens einen aminofunktionellen Asparaginsäureester als Härter mit optional wenigstens einem Füllstoff als erste Komponente und als zweite Komponente ein isocyanatfunktionelles Präpolymer enthält, wobei die Polymerkomponente der Polymerfolie ausgewählt ist aus thermoplastischen Polymeren und die Polymerfolie gegenüber dem ausgehärteten Gewebekleber-System eine Abzugskraft im Rollenschälversuch gemäß DIN EN 1464 - 2010 - 06 von weniger als 2,5 N/cm aufweist.
